# EUROPÄISCHE PATENTSCHRIFT

(11) **EP 1 165 152 B1**
(45) Veröffentlichungstag und Bekanntmachung des Hinweises auf die Patenterteilung: **30.06.2004**
(21) Anmeldenummer: 00917048.1
(22) Anmeldetag: 05.04.2000
(51) Int. Cl.: A61L 9/014, C09D 183/04, C08K 3/22

(54) **BESCHICHTUNG FÜR HAUSHALTSGERÄTE AUF SILANBASIS MIT DESODORISIERENDER WIRKUNG**
SILANE-BASED COATING WITH A DEODORIZING EFFECT FOR DOMESTIC APPLIANCES
REVETEMENT POUR APPAREILS MENAGERS A BASE DE SILANE AVEC EFFET DESODORISANT

(30) Priorität: 06.04.1999 DE 19915378
(43) Veröffentlichungstag der Anmeldung: 02.01.2002
(73) Patentinhaber: INSTITUT FÜR NEUE MATERIALIEN GEM. GMBH, 66123 Saarbrücken (DE); BSH Bosch und Siemens Hausgeräte GmbH, 81739 München (DE)
(72) Erfinder: BENTHIEN, Thomas, D-86899 Landsberg am Lech (DE); FABER, Stefan, D-66687 Wadern (DE); JONSCHKER, Gerhard, D-66583 Spiesen-Elversberg (DE); SEPEUR, Stefan, D-66787 Wadgassen-Schaffhausen (DE); SCHMIDT, Helmut, D-66130 Saarbrücken-Güdingen (DE); STÖSSEL, Philipp, D-66125 Saarbrücken (DE); JÖRDENS, Frank, D-83301 Traunreut (DE); SCHMIDMAYER, Gerhard, D-83093 Bad Endorf (DE)
(74) Vertreter: Barz, Peter
(86) Internationale Anmeldenummer: PCT/EP2000/003021
(87) Internationale Veröffentlichungsnummer: WO 2000/059555

(56) Entgegenhaltungen:
- EP-A- 0 459 003
- EP-A- 0 643 014
- EP-A- 0 842 967
- US-A- 5 624 667
- US-A- 5 924 292

## Beschreibung

Die Erfindung betrifft Haushaltsgeräte, die eine katalytische Zusammensetzung zum Zwecke der Desodorierung und Oxidation von organischen Komponenten und Kohlenstoff aufweisen.

EP-A-0604919 beschreibt ein Desodorierungsmittel, das Kompositteilchen umfasst, die eine Ceroxyverbindung und/oder eine Titanoxidverbindung enthalten. Das Desodorierungsmittel eignet sich zur Geruchsverminderung bei übelriechenden Gasen und für die Verwendung in Sanitärgegenständen.

EP-A-0842967 beschreibt einen Verbundwerkstoff, bei dem ein Substrat auf Basis von Glasfasern, Mineralfasern oder Holzwerkstoffen mit einem Nanokomposit in funktionellem Kontakt steht, wobei das Nanokomposit durch Oberflächenmodifizierung von kolloidalen anorganischen Partikeln mit einem oder mehreren Silanen erhalten wird. Als Beispiel für kolloidale anorganische Partikel wird z.B. TiO₂ genannt.

EP-A-0643014 beschreibt ein Desodorierungsmittel, bei dem Aktivkohle mit Metallsalzen imprägniert und thermisch behandelt wird, um mit Metalloxid belegte Aktivkohleteilchen zu erhalten, die katalytisches Oxidationsvermögen aufweisen. Diese werden mit Polymerteilchen gemischt, um schließlich als Formkörper in Kühlschränken desodorierend zu wirken.

US-A-5624667 beschreibt desodorierend wirkende TiO₂-Teilchen mit spezifischen Anteilen an Zink- oder Zink/Siliciumoxiden. EP-A-0459003 beschreibt Beschichtungszusammensetzungen aus Kondensaten, Füllstoffen und antimikrobiologisch und oxidierend wirkenden Metallsalzen von Ag, Co oder Zn. Die Beschichtungszusammensetzungen werden auf teilchenförmiges Material, z.B. Sand, aufgetragen und thermisch ausgehärtet. Die beschichteten Teilchen lassen sich als antimikrobiologisch wirksame Desodorantien im wässrigen Medium und in luftfiltern einsetzen.

Aufgabe der vorliegenden Erfindung ist die Bereitstellung von katalytischen Zusammensetzungen für Haushaltsgeräte, die in der Lage sind, Geruchsbelastungen in der Umgebung zu verringern oder zu beseitigen (Desodorierung) und die organische Komponenten oder Kohlenstoff oxidieren können.

Dieses Ziel wird überraschenderweise durch ein Haushaltsgerät mit einer katalytischen Zusammensetzung erreicht, die eine Beschichtung aus einer Beschichtungsmasse auf einem Substrat umfaßt und erhältlich ist durch Aufbringen der Beschichtungsmasse, umfassend (1) ein Polykondensat aus
(A) einem oder mehreren Silanen der allgemeinen Formel (I)

   RₐSiX₍₄₋ₐ₎ (I)

   worin die Reste R gleich oder verschieden sind und nicht hydrolysierbare Gruppen darstellen, die Reste X gleich oder verschieden sind und hydrolysierbare Gruppen oder Hydroxylgruppen bedeuten und a den Wert 0, 1, 2 oder 3 hat, wobei bei mindestens 50 Stoffmengen-% der Silane a größer 0 ist, oder einem davon abgeleiteten Oligomer,
(B) gegebenenfalls einer oder mehreren Verbindungen von glasbildenden Elementen,
und (2) Teilchen von einem oder mehreren katalytisch wirkenden Übergangsmetalloxiden oder Teilchen, die ein oder mehrere katalytisch wirkende Übergangsmetalloxide auf der Oberfläche aufweisen, wobei das Gewichtsverhältnis von Übergangsmetalloxid-Teilchen zu Polykondensat 10 : 1 bis 1 : 10 beträgt, auf das Substrat und thermisches Behandeln der aufgebrachten Beschichtungsmasse, wobei die katalytische Zusammensetzung Bestandteil des Haushaltsgeräts oder einer mit dem Haushaltsgerät verbundenen Einrichtung ist.

Bei den hydrolysierbaren Silanen (A) sind die hydrolysierbaren Gruppen X beispielsweise Wasserstoff oder Halogen (F, Cl, Br oder l), Alkoxy (vorzugsweise C₁₋₆-Alkoxy, wie z.B. Methoxy, Ethoxy, n-Propoxy, i-Propoxy und Butoxy), Aryloxy (vorzugsweise C₆₋₁₀-Aryloxy, wie z.B. Phenoxy), Acyloxy (vorzugsweise C₁₋₆-Acyloxy, wie z.B. Acetoxy oder Propionyloxy), Alkylcarbonyl (vorzugsweise C₂₋₇-Alkylcarbonyl, wie z.B. Acetyl), Amino, Monoalkylamino oder Dialkylamino mit vorzugsweise 1 bis 12, insbesondere 1 bis 6 Kohlenstoffatomen.

Bei den nicht hydrolysierbaren Resten R kann es sich um nicht hydrolysierbare Reste R¹ oder um eine funktionelle Gruppe tragende Reste R² handeln, wobei R¹ bevorzugt ist.

Der nicht hydrolysierbare Rest R¹ ist beispielsweise Alkyl (vorzugsweise C₁₋₈-Alkyl, wie Methyl, Ethyl, n-Propyl, Isopropyl, n-Butyl, s-Butyl und t-Butyl, Pentyl, Hexyl, Octyl oder Cyclohexyl), Alkenyl (vorzugsweise C₂₋₈-Alkenyl, wie z.B. Vinyl, 1-Propenyl, 2-Propenyl und Butenyl), Alkinyl (vorzugsweise C₂₋₈-Alkinyl, wie z.B. Acetylenyl und Propargyl) und Aryl (vorzugsweise C₈₋₁₀-Aryl, wie z.B. Phenyl und Naphthyl). Die genannten Reste R¹ und X können gegebenenfalls einen oder mehrere übliche Substituenten, wie z.B. Halogen oder Alkoxy, aufweisen.

Spezielle Beispiele für die funktionellen Gruppen des Restes R² sind die Epoxy-, Hydroxy-, Ether-, Amino-, Monoalkylamino-, Dialkylamino-, Amid-, Carboxy-, Vinyl-, Acryloxy-, Methacryloxy-, Cyano-, Halogen-, Aldehyd-, Alkylcarbonyl-, und Phosphorsäuregruppe. Diese funktionellen Gruppen sind über Alkylen-, Alkenylen- oder Arylen-Brückengruppen, die durch Sauerstoff- oder -NH-Gruppen unterbrochen sein können, an das Siliciumatom gebunden. Die genannten Brückengruppen leiten sich z.B. von den oben genannten Alkyl-, Alkenyl- oder Arylresten ab. Die Reste R² enthalten vorzugsweise 1 bis 18, insbesondere 1 bis 8 Kohlenstoffatome.

In einer bevorzugten Ausführungsform handelt es sich bei den Silanen (A) um eine Mischung aus
(A1) mindestens einem hydrolysierbaren Silan der allgemeinen Formel (II)

   SiX₄ (II)

   in der die Reste X gleich oder verschieden sind und hydrolysierbare Gruppen oder Hydroxylgruppen bedeuten, oder einem davon abgeleiteten Oligomer, und
(A2) mindestens einem Organosilan der allgemeinen Formel (III)

   R¹ ₐ₁R² ₐ₂SiX₍₄₋ₐ₁₋ₐ₂₎ (III)

   in der R¹ gleich oder voneinander verschieden sind und eine nicht hydrolysierbare Gruppe bedeuten, R² gleich oder voneinander verschieden sind und einen eine funktionelle Gruppe tragenden Rest bedeuten, X die vorstehende Bedeutung hat und a1 und a2 den Wert 0, 1, 2 oder 3 haben, wobei die Summe (a1+a2) den Wert 1, 2 oder 3 hat, oder einem davon abgeleiteten Oligomer
in einem Stoffmengenverhältnis (A1):(A2) von 5-50 : 50-95.

In der allgemeinen Formel (III) hat a1 vorzugsweise den Wert 1 oder 2, a2 vorzugsweise den Wert 0, 1 oder 2 und die Summe (a1+a2) vorzugsweise den Wert 1 oder 2.

Besonders bevorzugte hydrolysierbare Silane (A) bzw. (A1) sind Tetraalkoxysilane wie Tetraethoxysilan (TEOS). Besonders bevorzugte hydrolysierbare Silane (A) bzw. (A2) sind Alkyltrialkoxysilane, bevorzugt mit C₁-C₈-Alkyl, insbesondere Methyltriethoxysilan, Aryltrialkoxysilane, insbesondere Phenyltriethoxysilan, Dialkyldialkoxysilane, bevorzugt mit C₁-C₈-Alkyl, insbesondere Dimethyldiethoxysilan, und Diaryldialkoxysilane, insbesondere Diphenyldiethoxysilan. Silane mit funktionellen Gruppen (A) bzw. (A2) sind z. B. Epoxysilane wie 3-Glycidyloxypropyl-trimethoxysilan (GPTS) und Aminosilane wie 3-Aminopropyl-triethoxysilan und 3-(Aminoethylamino)-propyl-triethoxysilan (DIAMO).

In der Silankomponente (A) gemäß Formel (I) ist bei mindestens 50 Stoffmengen-% der Silane a größer 0, d.h., mindestens 50 Stoffmengen-% der Silane enthalten mindestens eine nicht hydrolysierbare Gruppe R. Vorzugsweise enthält die Silankomponente (A) 50 bis 95 Stoffmengen-% Silane mit mindestens einer nicht hydrolysierbaren Gruppe R. Bezüglich der Formeln (II) und (III) ist das bevorzugte Stoffmengenverhältnis des hydrolysierbaren Silans (A1) zu dem Organosilan (A2) im Polykondensat 5 bis 50 : 50 bis 95, vorzugsweise 1:1 bis 1:6 und besonders bevorzugt 1:3 bis 1:5. Ein besonderes günstiges Stoffmengenverhältnis ist 1:4.

Die Eventualkomponente (B) stellt glasbildende Elemente dar, die vorzugsweise im Reaktionsmedium löslich oder dispergierbar sind. Verwendbar sind z.B. Verbindungen (Halogenide, Alkoxide, Carboxylate, Chelate, etc.) von Lithium, Natrium, Kalium, Rubidium, Cäsium, Beryllium, Magnesium, Calcium, Strontium, Barium, Bor, Aluminium, Titan, Zirkon, Zinn, Zink oder Vanadium.

Zur Herstellung des Polykondensats (1) werden die Ausgangskomponenten (A) und gegebenenfalls (B) hydrolysiert und kondensiert. Die Hydrolyse und Kondensation wird entweder in Abwesenheit eines Lösungsmittel oder vorzugsweise in einem wäßrigen oder wäßrig/organischen Reaktionsmedium, gegebenenfalls in Gegenwart eines sauren oder basischen Kondensationskatalysators wie HCl, HNO₃ oder NH₃ durchgeführt. Bevorzugt erfolgt die Hydrolyse und Kondensation in Gegenwart einer wäßrigen Säure. Die wäßrigen Säuren werden bevorzugt in einem Konzentrationsbereich von 0,1 N bis 10,0 N eingesetzt. Bevorzugt eingesetzte Säuren sind Salz-, Salpeter-, Phosphor- und Essigsäure.

Daneben können bei der Herstellung des Polykondensats die nachstehend aufgeführten anorganischen Partikel zugegeben werden. Bei der Herstellung können bevorzugt nanoskalige anorganische Partikel, insbesondere in Form eines Sols, zugegeben werden. Beispielsweise können Kieselsole im Sol als hydrolytisch aktive Verbindung wirken. Hierfür eignen sich handelsübliche Kieselsole, wie beispielsweise die Levasile®, Kieselsole der Bayer AG.

Bei Einsatz eines flüssigen Reaktionsmediums sind die Ausgangskomponenten in dem Reaktionsmedium löslich. Als organische Lösungsmittel eignen sich insbesondere mit Wasser mischbare Lösungsmittel, wie z.B. ein- oder mehrwertige aliphatische Alkohole, aber auch aliphatische oder aromatische Kohlenwasserstoffe, z. B. mit 5 bis 20 Kohlenstoffatomen, Ether, Ester, Ketone, Amide und Alkylamide.

Vorzugsweise erfolgt die Hydrolyse und Polykondensation unter den Bedingungen des Sol-Gel-Prozesses, wobei das Reaktionsgemisch im viskosen Sol-Zustand zum Beschichten des Substrats verwendet wird.

Gegebenenfalls wird die Hydrolyse und Polykondensation in Gegenwart eines Komplexbildners durchgeführt, z.B. Nitraten, ß-Dicarbonylverbindungen (z.B. Acetylacetonaten oder Acetessigsäurester), Carbonsäuren (z.B. Methacrylsäure) oder Carboxylaten (z.B. Acetat, Citrat oder Glykolat), Betainen, Diolen, Diaminen (z.B. DIAMO) oder Kronenether.

Das Verhältnis der hydrolytisch aktiven Komponenten zu den hydrolysierbaren Silanen (und gegebenenfalls den glasbildenden Elementen) kann durch den R_{OR}-Wert gekennzeichnet werden. Der R_{OR}-Wert stellt das Stoffmengenverhältnis von Wasser aus den hydrolytisch aktiven Komponenten (Wasser, wäßrige Säure, Kieselsol usw.) zu den vorstehend aufgeführten hydrolysierbaren Gruppen X aus den Silankomponenten (und gegebenenfalls den entsprechenden hydrolysierbaren Gruppen der glasbildenden Elementen) dar. Das erhaltene Sol besitzt beispielsweise einen R_{OR}-Wert von 0,1 bis 10 und bevorzugt von 0,2 bis 2.

Das erhaltene Polykondensat wird bevorzugt in Form eines Sols mit Teilchen von einem oder mehreren Übergangsmetalloxiden vermischt, wobei das Verhältnis von Übergangsmetalloxid-Teilchen zu Polykondensat 10 : 1 bis 1 : 10, bevorzugt 10 : 1 bis 1 : 1 und besonders bevorzugt 10 : 1 bis 2 : 1 beträgt. Bei diesem Verhältnis werden für das Polykondensat mit Ausnahme von gegebenenfalls hinzugefügtem organischem Lösungsmittel alle zur Herstellung des Polykondensats hinzugegebenen Komponenten (insbesondere die anorganischen Partikel zur Herstellung des Kondensats) berücksichtigt.

Der durchschnittliche Teilchendurchmesser der eingesetzten Übergangsmetalloxide liegt beispielsweise in einem Bereich von 10 nm bis 20 µm. Bei beschichteten Substraten, die zur Geruchsverbesserung eingesetzt werden sollen, werden bevorzugt Übergangsmetalloxid-Teilchen mit einem durchschnittlichen Teilchendurchmesser von 1 bis 20 µm eingesetzt.

Die Teilchen bestehen im wesentlichen beziehungsweise bevorzugt vollständig aus Übergangsmetalloxid. Die Übergangsmetalloxid-Teilchen können aus einem Übergangsmetalloxid oder aus Übergangsmetalloxid-Mischungen bestehen. Bei den bevorzugt eingesetzten Übergangsmetalloxid-Mischungen werden bevorzugt verschiedene Übergangsmetalloxidpulver miteinander vermengt, so daß Teilchen aus verschiedenen Übergangsmetalloxiden vorliegen. Selbstverständlich können auch Teilchen, die verschiedene Übergangsmetalloxide enthalten, eingesetzt werden.

Insbesondere bei der Verwendung für Oxidationszwecke können aber neben den im wesentlichen aus Übergangsmetalloxiden bestehenden Teilchen, auch teilweise oder vollständig Teilchen eingesetzt werden, die an der Oberfläche die nachstehend aufgeführten Übergangsmetalloxide aufweisen, ansonsten aber aus einem anderen Material' bestehen. Die Übergangsmetalloxid-Teilchen bestehen dann aus Teilchen eines Materials, das vorzugsweise aus einem der nachstehend für die anorganischen Partikel genannten Materialien gewählt ist, das an der Oberfläche mit einem oder mehreren Übergangsmetalloxiden beschichtet ist. Vorzugsweise sind diese Teilchen an der Oberfläche vollständig mit den Übergangsmetalloxiden beschichtet. Diese Teilchen werden bei dem Gewichtsverhältnis von Übergangsmetalloxid-Teilchen zu Polykondensat als Ganzes als Übergangsmetalloxid-Teilchen berücksichtigt. Hierbei handelt es sich insbesondere um die nachstehend aufgeführten Partikel im Mikrometerbereich, die an der Oberfläche mit Übergangsmetalloxiden versehen beziehungsweise imprägniert sind.

Bei den Übergangsmetalloxiden handelt es sich um katalytisch wirkende Übergangsmetalloxide, die desodorierende und/oder oxidierende Eigenschaften aufweisen. Unter den Übergangsmetallen werden wie üblich die Elemente der Nebengruppen I bis VIII des Periodensystems und die Lanthaniden- und Actiniden-Elemente verstanden. Das Übergangsmetalloxid wird besonders bevorzugt ausgewählt aus den Oxiden der Metalle La, Ce, Ti, Zr, V, Cr, Mo, W, Mn, Fe, Co, Ni, Cu, Ag und Zn oder aus Mischungen dieser Metalloxide. Bevorzugt eingesetzt werden Übergangsmetalloxid-Mischungen, wobei Mischungen der Oxide von Mn und Ce mit einem oder zwei weiteren Übergangsmetallen, wie Mischungen der Oxide von Mn/Co/Ce, Mn/Cu/Ce, Mn/Ni/Ce, Mn/Fe/Ce oder Mn/Co/Ni/Ce, besonders bevorzugt sind. Eine weiter bevorzugte Übergangsmetalloxid-Mischung ist eine Mischung der Oxide von Cu/V/La. Es können auch Mischoxide der vorgenannten Übergangsmetalle eingesetzt werden.

In den Übergangsmetalloxid-Mischungen sind folgende Mengen der entsprechenden Metalloxide in der Metalloxid-Mischung bevorzugt: Ce: 1-70 Gew.%, V: 5-70 Gew.%, Mn: 20-95 Gew.%, Fe: 20-95 Gew.%, Co: 1-50 Gew.%, Ni: 1-50 Gew.%, Cu: 1-95 Gew.%.

Konkrete Beispiele für Übergangsmetalloxide sind MnO₂ (Pyrolusit), γ-MnO₂, Co₃O₄, Co₂O₃, CoO und CeO₂. Natürlich kann auch jedes andere geeignete Übergangsmetalloxid eingesetzt werden.

Die BET-Oberfläche der eingesetzten Teilchen liegt beispielsweise in einem Bereich von 1 bis 100 m²/g.

Neben den Übergangsmetalloxid-Teilchen können in der Beschichtungsmasse zusätzlich Co-Katalysatoren, z. B. in Mengen von 1 bis 5 Gew.-%, bezogen auf die Übergangsmetalloxid-Teilchen, eingesetzt werden. Geeignete Co-Katalysatoren sind etwa K-, Mg-, Ca-, Ba- und Sr-Salze sowie Al- und Sn-Oxid. Als Salze eignen sich beispielsweise die entsprechenden Halogenide, Hydroxide, Nitrate, Carbonate, Phosphate oder Carbonate. Die Zugabe kann z.B. durch Mischen des Co-Katalysators mit den Übergangsmetalloxid-Teilchen oder mit den Mischungen der Übergangsmetalloxid-Teilchen vor Zugabe zum Polykondensat oder durch separate Zugabe des Co-Katalysators in die Beschichtungsmasse erfolgen. Im ersteren Fall werden bevorzugt Pulver eingesetzt und im letzteren Fall verwendet man bevorzugt leicht lösliche Salze des Co-Katalysators.

In der Beschichtungsmasse können auch anorganische Partikel enthalten sein, die bei der Herstellung des Polykondensats oder der Beschichtungsmasse oder danach zugegeben werden können. Dabei kann es sich um nanoskalige anorganische Partikel oder um Partikel im Mikrometerbereich handeln. Es können auch Partikel beider Größenordnungen zugegeben werden, wobei die Partikel im Mikrometerbereich insbesondere bei Verwendung der katalytischen Zusammensetzung zur Oxidation von organischen Komponenten oder Kohlenstoff eingesetzt werden.

Die anorganischen Partikel können aus beliebigen Materialien bestehen, vorzugsweise handelt es sich um Oxide. Bevorzugte Oxide sind Oxide von Si und Al (insbesondere Boehmit). Die Partikel können z. B. in Form von Pulvern oder, insbesondere die nanoskaligen, in Form von Solen zugegeben werden.

Die nanoskaligen anorganischen Partikel besitzen vorzugsweise eine durchschnittliche Teilchengröße bis zu 300, insbesondere bis zu 100 nm und besonders bevorzugt bis zu 50 nm. Die Partikel können in kolloidaler Form zugegeben werden. Dabei kann es sich um Sole oder dispergierbare Pulver handeln. Spezielle Beispiele für nanoskalige anorganische Partikel sind SiO₂, Al₂O₃, SnO₂, Eisenoxide oder Kohlenstoff (Ruß und Graphit), insbesondere SiO₂. Ganz bevorzugt werden Kieselsole als nanoskalige anorganische Teilchen eingesetzt.

Insbesondere wenn die katalytischen Zusammensetzungen als oxidativ wirkende Zusammensetzungen eingesetzt werden sollen, können zur Beschichtungsmasse auch anorganische Partikel im Mikrometerbereich zugegeben werden. Sie dienen zur Strukturierung der Beschichtung und zur Erzeugung von Hohlräumen. Diese Partikel besitzen einen durchschnittlichen Partikeldurchmesser von beispielsweise 1 bis 500, bevorzugt 10 bis 300 µm. Es handelt sich dabei bevorzugt um oxid- und/oder hydroxylhaltige Verbindungen der Elemente der III und IV Hauptgruppe wie Aluminium- oder Siliciumoxide. Sie können aktiviert sein. Als Beispiele können Kieselgur, Aluminiumoxid 90, Kieselgel 40 oder Kieselgel 60, hergestellt von der Firma Merck, genannt werden.

Die oben genannten anorganischen Partikel im Mikrometerbereich können vor dem Einsatz mit Metallsalzen oder Mischungen von Metallsalzen, z. B. Chloriden, Phosphaten, Formiaten, Nitraten oder Acetaten, getränkt und anschließend bei erhöhten Temperaturen getempert werden, um katalytisch aktive Metalloxide auf der Oberfläche zu erzeugen. Bevorzugt werden Metallnitrate oder Metallacetate eingesetzt, da die Anionen bei der Behandlung im verwendeten Temperaturbereich flüchtige Produkte bilden. Als Metalle werden die für die Übergangsmetalloxid-Teilchen genannten Übergangsmetalle eingesetzt. Man erhält dann Partikel, die an der Oberfläche mit Übergangsmetalloxiden versehen sind, und die erfindungsgemäß als Übergangsmetalloxid-Teilchen eingesetzt werden, und beim Gewichtsverhältnis von Übergangsmetalloxid-Teilchen zu Polykondensat als Ganzes berücksichtigt werden.

Die Beschichtungsmasse kann auch weitere Additive enthalten. Es können z.B. Additive verwendet werden, die sich zur Viskositätseinstellung und/oder insbesondere zur Erzeugung von Hohlräumen bei der thermischen Behandlung der Beschichtungsmassen eignen. Hierfür können z.B. übliche Verdickungsmittel eingesetzt werden. Konkrete Beispiele sind Cellulose-Derivate, z. B. Hydroxypropylcellulose, Stärke, modifizierte Stärke, Polyvinylalkohol und Glycole, z. B. Polyethylenglycol. Bevorzugt werden Cellulose-Derivate, insbesondere Hydroxypropylcellulose verwendet. Daneben können auch die in katalytischen Zusammensetzungen üblichen Additive wie Pigmente (z.B. Schwarzpigmente) eingesetzt werden.

Die Viskosität des mit den Übergangsmetalloxid-Teilchen vermischten Sols kann gegebenenfalls noch durch Entfernen oder Zugabe eines Lösungsmittels, z. B. eines der vorstehend genannten, eingestellt werden. Es ist in dieser Form üblicherweise auch lange lagerfähig. Gegebenenfalls kann es durch Zugabe von Wasser oder wäßriger Säure aktiviert werden, wobei die Beschichtungsmasse dann bevorzugt innerhalb eines Monats eingesetzt wird.

Die Beschichtungsmasse wird nach üblichen Beschichtungsmethoden auf das Substrat aufgebracht. Anwendbare Techniken sind z.B. das Tauchen, Gießen, Schleudern, Aufsprühen oder Aufstreichen.

Geeignete Substrate sind z.B. solche aus Metallen wie Edelstahl, Stahl, Kupfer, Messing und Aluminium; Metalloxiden, Gläsern wie Floatglas, Borosilikatglas, Bleikristall oder Kieselglas; Glaskeramiken, und Keramiken wie Al₂O₃, ZrO₂, SiO₂-Mischoxiden oder auch Email, aber auch poröse Träger wie z.B. poröse Keramiken. Die Form der Substrate ist beliebig. Es kann sich um ebene oder strukturierte Substrate handeln. Besonders geeignet sind Substrate in Form von Geflechten, Wabenkörpern oder Netzen, etwa Drahtgeflechte, beispielsweise Stahldrahtgeflechte, keramische Wabenkörper oder Drahtnetze.

Vor dem Auftrag der Beschichtungsmasse können die Substrate vorbehandelt werden. Beispielsweise werden sie einer Reinigung, z. B. mit handelsüblichen alkalischen Reinigern, unterworfen. Ebenfalls kann z.B. durch das Tempern von Stahlsubstraten und die Ausbildung von Chromoxidwhiskem an der Oberfläche eine wesentlich verbesserte Haftung des Beschichtungsmaterials auf Stahlsubstraten erreicht werden.

Der erhaltene Überzug wird gegebenenfalls vorgetrocknet und dann thermisch behandelt. Dies kann bei Temperaturen von 200°C bis 700°C, vorzugsweise 300°C bis 400°C, erfolgen. Die thermische Behandlung kann an der Luft oder in einem Inertgas wie Stickstoff oder Argon durchgeführt werden. Die Wärmebehandlung kann gegebenenfalls auch durch IR- oder Laser-Strahlung erfolgen. Bei der thermischen Behandlung kann es z.B. zur Trocknung, Härtung oder Verfestigung bzw. Verdichtung der Beschichtungsmasse kommen.

Die Beschichtung wird bevorzugt so ausgeführt, daß Schichtdicken von 0,01 bis 500 µm, bevorzugt von 1 bis 500 µm, erhalten werden. Werden die katalytischen Zusammensetzungen zum Zwecke der Desodorierung eingesetzt, sind Schichtdicken von 30 bis 100 µm, insbesondere 25 bis 75 µm, bevorzugt. Werden die katalytischen Zusammensetzungen als oxidativ wirksame Oberflächen eingesetzt, sind bei Verwendung von Übergangsmetalloxiden mit einer durchschnittlichen Teilchengröße von unter 200 nm Schichtdicken von 1 bis 10 µm geeignet. Die als oxidativ wirksame Oberflächen dienenden katalytischen Zusammensetzungen, die zusätzlich anorganische Partikel im Mikrometerbereich enthalten, weisen bevorzugt Schichtdicken von 100 bis 400 µm auf.

Die erfindungsgemäßen katalytischen Zusammensetzungen können eine poröse oder eine nicht poröse Beschichtung aufweisen. Vorzugsweise weisen die katalytischen Zusammensetzungen poröse Beschichtungen auf. Bei den Poren kann es sich dabei um mikroskopisch sichtbare Hohlräume an der Oberfläche und/oder um feinere Mikroporen handeln. Die unter dem Mikroskop auf der Oberfläche sichtbaren Hohlräume zeigen etwa eine globuläre Morphologie (Halbkugeln) und ihr Durchmesser beträgt etwa 1 bis 5 µm. Ihre Ausdehnung und Form im Innern der Schicht läßt sich mikroskopisch nicht feststellen. Die Bestimmung der BET-Oberflächen von bevorzugten Ausführungsformen weist darauf hin, daß alternativ oder zusätzlich feinere Mikroporen darin vorhanden sind.

Die erfindungsgemäße katalytische Zusammensetzung wirkt desodorierend, d.h. durch Substanzen verursachte Geruchsbelastungen können verringert oder ganz vermieden werden. Die desodorierende Wirkung wird insbesondere bei Temperaturen über 150°C, z.B. bei Temperaturen von 150 bis 500°C, bevorzugt 200 bis 350°C, festgestellt. Die geruchsbelastete Luft wird dabei an der katalytischen Zusammensetzung bei erhöhten Temperaturen vorbeigeleitet. Dabei werden in der Luft befindliche Substanzen abgebaut.

Die katalytische Zusammensetzung ist auch in der Lage organische Komponenten oder Kohlenstoff, z.B. Ruß oder Graphit, die sich beispielsweise auf der Oberfläche der katalytischen Zusammensetzung befinden, zu oxidieren. Die oxidierende Wirkung wird insbesondere bei den vorstehend ausgeführten Temperaturbereichen festgestellt.

Die katalytische Zusammensetzungen werden dabei bevorzugt so eingesetzt, daß sie sich direkt auf irgendeiner Oberfläche des jeweiligen Haushaltsgerätes befinden, wobei dann diese Oberfläche als Substrat dient, oder die katalytische Zusammensetzung ist Bestandteil einer gegebenenfalls über eine Anschlußleitung verbundenen Zusatzeinrichtung innerhalb oder in der Nähe des Haushaltsgerätes. Als Oberfläche des Haushaltsgerätes kommt z.B. ein Gehäuseteil oder eine Innenwand, insbesondere aus Metall, in Frage. Bei der Zusatzeinrichtung kann es sich z.B. um eine Anordnung handeln, durch die die Abluft des Haushaltsgeräts geführt wird. Diese Zusatzeinrichtung kann neben der katalytischen Zusammensetzung z.B. eine Heiz- oder Belüftungseinrichtung aufweisen.

Auf diese Weise ist es auf einfache Weise möglich, den Geruchsbelastungen oder Oberflächenverschmutzungen, die in den Haushalten bzw. den Haushaltsgeräten auftreten können, entgegenzuwirken. Bei den erfindungsgemäß eingesetzten Haushaltsgeräten handelt es sich um die üblichen im Haushalt verwendeten Gegenstände. Beispiele sind Herde, Kochmulden, Dunstabzugshauben und Friteusen. Bevorzugte Einsatzgebiete sind Elektro- und Gasherde, Dunstabzugshauben und/oder Friteusen.

### BEISPIELE

### A. Herstellung der Silansole

### Silansol 1: MTKS-Sol, R_{OR} = 0,4

Eine Mischung aus 1069,86 g (6,0 mol) Methyltriethoxysilan und 312,48 g (1,5 mol) Tetraethoxysilan wird in zwei Portionen (Portion 1 und Portion 2) gleichen Gewichts geteilt.
Zu Portion 1 gibt man unter gutem Rühren 246,84 g Kieselsol (Levasil 300/30, wäßrig, basisch, Bayer AG). Nachdem sich eine Emulsion gebildet hat (ca. 30 s) gibt man 5,60 g 36 Gew.-%ige HCl zu. Nach kurzem Rühren (30-50 s) wird die Reaktionsmischung unter Erwärmen klar. Zu dieser Reaktionsmischung gibt man die Portion 2 in einem Zuge schnell zu. Nach kurzer Zeit trübt sich die Reaktionsmischung durch einen farblosen Niederschlag (NaCl). Anschließend rührt man noch 15 min unter Kühlung in einem Eisbad nach. Man läßt das Silanhydrolysat 12 h bei Raumtemperatur stehen, dekantiert vom sedimentierten Feststoff ab, und erhält so das gebrauchsfähige MTKS-Sol.

### Silansol 2: MDKS-Sol, ROR = 0,2

Eine Mischung aus 356,62 g (2,0 mol) Methyltriethoxysilan und 74,14 g (0,5 mol) Dimethyldiethoxysilan wird unter gutem Rühren gleichzeitig mit 35,10 g Kieselsol (Levasil 300/30, wäßrig, basisch, Bayer AG) und 1,10 g 36 Gew.-%iger HCl versetzt. Nach kurzem Rühren (30-50 s) wird die Reaktionsmischung unter Erwärmen klar. Nach kurzer Zeit trübt sich die Reaktionsmischung durch einen farblosen Niederschlag (NaCl). Anschließend rührt man noch 15 min unter Kühlung in einem Eisbad nach. Man läßt das Silanhydrolysat 12 h bei Raumtemperatur stehen, dekantiert vom sedimentierten Feststoff ab, und erhält so das gebrauchsfähige MDKS-Sol.

### Silansol 3: MPTKS-Sol, ROR = 0,4

Eine Mischung aus 11,59 g (0,065 mol) Methyltriethoxysilan, 3,61 g (0,015 mol) Phenyltriethoxysilan und 4,17 g (0,020 mol) Tetraethoxysilan wird unter gutem Rühren gleichzeitig mit 3,29 g Kieselsol (Levasil 300/30, wäßrig, basisch, Bayer AG) und 0,13 g 36 Gew.-%iger HCl versetzt. Nach kurzem Rühren (30-50 s) wird die Reaktionsmischung unter Erwärmen klar. Nach kurzer Zeit trübt sich die Reaktionsmischung durch einen farblosen Niederschlag (NaCl). Anschließend rührt man noch 15 min unter Kühlung in einem Eisbad nach. Man läßt das Silanhydrolysat 12 h bei Raumtemperatur stehen, dekantiert vom sedimentierten Feststoff ab, und erhält so das gebrauchsfähige MPTKS-Sol.

### Silansol 4: MPrTKS-Sol, ROR = 0,4

Eine Mischung aus 15,00 g (0,084 mol) Methyltriethoxysilan, 14,95 g (0,091 mol) *n*-Propyltrimethoxysilan und 8,96 g (0,043 mol) Tetraethoxysilan wird unter gutem Rühren gleichzeitig mit 7,00 g Kieselsol (Levasil 300/30, wäßrig, basisch, Bayer AG) und 0,23 g 32 Gew.-%iger HCl versetzt. Nach kurzem Rühren (30-50 s) wird die Reaktionsmischung unter Erwärmen klar. Nach kurzer Zeit trübt sich die Reaktionsmischung durch einen farblosen Niederschlag (NaCl). Anschließend rührt man noch 15 min unter Kühlung in einem Eisbad nach. Man läßt das Silanhydrolysat 12 h bei Raumtemperatur stehen, dekantiert vom sedimentierten Feststoff ab, und erhält so das gebrauchsfähige MPrTKS-Sol.

### Silansol 5: MD-Sol, ROR = 0,4

Eine Mischung aus 35,66 g (0,2 mol) Methyltriethoxysilan und 7,41 g (0,05 mol) Dimethyldiethoxysilan wird mit 5,04 g 0,1 N HCl unter gutem Rühren versetzt. Nach kurzem Rühren (30-50 s) wird die Reaktionsmischung unter Erwärmen klar. Man läßt das Silanhydrolysat 12h bei Raumtemperatur stehen und erhält so das gebrauchsfähige MD-Sol.

### B. Herstellung der Katalysatormischungen

Als Katalysatormischungen werden Mischungen kommerzieller Übergangsmetalloxid-Pulver der Fa. Ferro oder Aldrich verwendet:
- MnO₂ :: Pulver der Fa. Ferro, überwiegend MnO₂ (Pyrolusit), wenig γ-MnO₂ und wenig MnO₂
- Co_{y}Oₓ :: Pulver der Fa. Ferro, überwiegend Co₃O₄, sehr wenig CoO

### Katalysatormischung 1: Mn/Co/Ce

Die Katalysatormischung 1 wird durch inniges Mischen von 800,00 g MnO₂, 100,00 g Co_{y}Oₓ und 100,00 g CeO₂ hergestellt.

### Katalysatormischung 2: Mn/Co/Ce

Die Katalysatormischung 2 wird durch inniges Mischen von 800,00 g MnO₂, 150,00 g Co_{y}Oₓ und 50,00 g CeO₂ hergestellt.

### Katalysatormischung 3: Mn/Cu/Ce

Die Katalysatormischung 3 wird durch inniges Mischen von 650,00 g MnO₂, 300,00g Cu₂O und 50,00 g CeO₂ hergestellt.

### Katalysatormischung 4: Mn/Co/Ni/Ce

Die Katalysatormischung 4 wird durch inniges Mischen von 700,00 g MnO₂, 100,00 g Co_{y}Oₓ, 150,00 g NiO und 50,00 g CeO₂ hergestellt.

### C. Herstellung der Beschichtungsmassen

### Beispiel 1

1000,00 g Katalysatormischung 1 werden mit 300,00 g Silansol 1 und 233,33 g Ethanol 2 h bei Raumtemperatur gerührt. Anschließend fügt man zur Aktivierung (Erhöhung des R_{OR}-Werts von 0,4 auf 0,8) 32,35 g 10 Gew.-%ige wäßrige Salzsäure zu, rührt mindestens 2 h bei Raumtemperatur nach, und erhält so die gebrauchsfertige Beschichtungssuspension.

### Beispiel 2

1000,00 g Katalysatormischung 2 werden mit 200,00 g Silansol 1 und 350,00 g Ethanol 2 h bei Raumtemperatur gerührt. Anschließend fügt man zur Aktivierung (Erhöhung des R_{OR}-Werts von 0,4 auf 0,8) 23,49 g 10 Gew.-%ige wäßrige Salzsäure zu, rührt mindestens 2 h bei Raumtemperatur nach, und erhält so die gebrauchsfertige Beschichtungssuspension.

### Beispiel 3

1000,00 g Katalysatormischung 3 werden mit 400,00 g Silansol 2 und 185,00 g Ethanol 1 h bei Raumtemperatur gerührt. Anschließend fügt man zur Aktivierung (Erhöhung des R_{OR}-Werts von 0,2 auf 0,6) 47,97 g 10 Gew.-%ige wäßrige Salzsäure zu, rührt mindestens 4 h bei Raumtemperatur nach, und erhält so die gebrauchsfertige Beschichtungssuspension.

### Beispiel 4

100,00 g Katalysatormischung 3 werden mit 18,00 g Silansol 3 und 25,00 g Ethanol 1 h bei Raumtemperatur gerührt. Anschließend fügt man zur Aktivierung (Erhöhung des R_{OR}-Werts von 0,4 auf 0,7) 1,52 g 10 Gew.-%ige wäßrige Salzsäure zu, rührt mindestens 2 h bei Raumtemperatur nach, und erhält so die gebrauchsfertige Beschichtungssuspension.

### Beispiel 5

100,00 g Katalysatormischung 4 werden mit 40,00 g Silansol 5 und 11,00 g Ethanol 1 h bei Raumtemperatur gerührt. Anschließend fügt man zur Aktivierung (Erhöhung des R_{OR}-Werts von 0,4 auf 0,8) 4,66 g 10 Gew.-%ige wäßrige Salzsäure zu, rührt mindestens 2 h bei Raumtemperatur nach, und erhält so die gebrauchsfertige Beschichtungssuspension.

### D. Beschichtung und thermische Behandlung (insbesondere für Desodorierungszwecke)

Als Trägermaterial werden Stahldrahtgeflechte (Durchmesser ca. 5 cm, Höhe ca. 1 cm) oder keramische Wabenkörper verwendet. Die Stahlgeflechte werden zunächst mit einem kommerziellen, alkalischen Reiniger entfettet, anschließend mit deionisiertem Wasser gut nachgespült, und dann bei Raumtemperatur getrocknet. Die trockenen Stahlgeflechte werden anschließend 1h bei 500°C getempert.

Die Beschichtung erfolgt durch Tränken der Stahldrahtgeflechte oder der keramischen Wabenkörper in einer der unter Abschnitt C beschriebenen Beschichtungsmassen (Beschichtungssuspensionen). Die überschüssige Beschichtungssuspension wird durch Durchblasen mit Preßluft entfernt. Nach Trocknung bei Raumtemperatur (2 h) erfolgt die Verfestigung der Beschichtung durch thermische Behandlung. Die beschichteten Substrate wurden dazu innerhalb von 1 h von Raumtemperatur auf 300°-400°C aufgeheizt, 1 h Stunde bei 300°-400°C gehalten, und anschließend während 6 h auf Raumtemperatur abgekühlt.

Alternativ kann die thermische Behandlung auch durch direktes Einstellen der getrockneten, beschichteten Substrate in einen auf 300°-400°C vortemperierten Ofen, und schnelles Abkühlen der heißen Substrate auf Raumtemperatur während einiger Minuten erfolgen.

Die Schichtdicken der thermisch verfestigten Schichten liegen typischerweise im Bereich von 25-75 µm. Die Schichtdicken können z.B. zum einen durch die Viskosität der Beschichtungssuspension (diese kann z.B. durch Zugabe einer geeigneten Menge an Ethanol eingestellt werden), zum anderen durch den Druck der Preßluft, bzw. die Einwirkzeit der Preßluft beim Entfernen der überschüssigen Beschichtungssuspension, eingestellt werden.

### E. Katalytische Zusammensetzung 1 (insbesondere für die Oxidation)

### E.1 Darstellung eines Mn/Cu/Ce - Katalysators auf einem Aluminiumoxid-Träger

40,47 g (0,141 mol) Mn(NO₃)₂ · 6 H₂O , 11,63 g (0,050 mol) Cu(NO₃)₂ · 3 H₂O und 15,20 g (0,035 mol) Ce(NO₃)₃ · 6 H₂O werden in einer Mischung aus 30,00 g Ethanol und 30,00 g Wasser bei 50°C gelöst. Zu dieser Lösung gibt man 100,00 g Aluminiumoxid 90 (aktiv, sauer (alternativ kann auch neutrales oder basisches eingesetzt werden), Partikelgröße 63-200 µm, Fa. Merck) und dampft das Lösungsmittelgemisch unter Rühren bei 50-70°C während 3 h ab. Das mit den Metallnitraten imprägnierte Aluminiumoxid wird anschließend 1h bei 500°C getempert. Analog können auch die entsprechenden molaren Mengen an Metallacetaten eingesetzt oder anstelle von Aluminium 90 die ebenfalls von der Fa. Merck hergestellten Kieselgel 40, Partikelgröße 63-200 µm, Kieselgel 60, Partikelgröße 63-200 µm oder Kieselgur, Partikelgröße ca. 100 µm, verwendet werden.

### E.2 Beschichtungsmasse

150,00 g des vorstehend beschriebenen Mn/Cu/Ce-Katalysators (E.1) auf einem Aluminiumoxid-Träger und 50,00 g Katalysatormischung 1 werden innig gemischt. Zu dieser Mischung fügt man unter Rühren 100,00 g einer 5 Gew.-%igen Lösung von Hydroxypropylcellulose in Ethanol. 140,00 g Silansol 2 werden zur Aktivierung (Erhöhung des R_{OR}- Werts von 0,2 auf 0,8) unter Rühren mit 22,67 g (1,26 mol) Wasser versetzt und 30 min bei Raumtemperatur gerührt. Das aktivierte MDKS-Sol wird unter Rühren bei Raumtemperatur zur oben beschriebenen Mischung aus Mn/Cu/Ce-Katalysator, Katalysatormischung 1 und Hydroxypropylcellulose in Ethanol gefügt und anschließend 15 min bei Raumtemperatur gerührt, um die gebrauchsfertige Beschichtungsmasse zu erhalten.

### E.3 Beschichtung und thermische Verfestigung

Als Trägermaterial werden Stahlsubstrate (Bleche 10 x 10 cm) verwendet. Die Stahlsubstrate werden zunächst mit einem kommerziellen, alkalischen Reiniger entfettet, anschließend mit deionisiertem Wasser gut nachgespült, und dann bei Raumtemperatur getrocknet. Die trockenen Stahlsubstrate können anschließend 1 h bei 500°C getempert werden.

Die gereinigten oder die gereinigten und getemperten Stahlsubstrate werden mit der Beschichtungsmasse geflutet. Die beschichteten Stahlsubstrate werden 1 h bei Raumtemperatur getrocknet, anschließend innerhalb von 1 h von Raumtemperatur auf 500°C aufgeheizt, 1 h bei 500°C gehalten, und anschließend innerhalb von 6 h auf Raumtemperatur abgekühlt.

Die Schichtdicken der thermisch verfestigten Schichten liegen typischerweise im Bereich von 150-400 µm, je nach verwendetem Trägermaterial und verwendeter Menge an Beschichtungsmasse.

### F. Bewertung

### Verfahren zur Bestimmung der desodorierenden Wirkung

In einen auf 300°C vorgewärmten Umluftofen (Temperatur am Katalysator ca. 300°C, Substrat: Stahldrahtgeflechte) werden nacheinander ca. 100 mg der Testsubstanzen:
Pyrazin, Thiazol, Maltol, Vanillin und 2,4-Decadienal
eingebracht.

Die Testsubstanzen verdampfen im heißen Umluftofen, die Dämpfe werden über den Umluftstrom als Abgase (Abgasstrom: 0.5-1.2 l/s) durch einen Auslaßstutzen ohne Katalysator und einen Auslaßstutzen mit Katalysator zu einem nachgeschalteten Probensammler geleitet. Die gesammelten Proben werden mit Hilfe der GC-MS-Spektroskopie analysiert. Aus den Spektren werden Abbauraten für die Testsubstanzen im Abgasstrom der über den Katalysator läuft, im Vergleich zum Abgasstrom der nicht über einen Katalysator läuft, ermittelt (Prinzip: Relativmessung an einem experimentellen Aufbau). Die Abbauraten sind nachfolgend in % angegeben.

| Katalysator | Pyrazin | Thiazol | Maltol | Vanilin | Decadienal |
|---|---|---|---|---|---|
| Pd/Pt-Kat(*1) | 0 | 0 | 90 | 90 | - |
| (*2) | 83 | 88 | 73 | 78 | 65 |
| (*3) | 69 | 56 | 74 | 70 | - |

| | | | | | |
|---|---|---|---|---|---|
| (*1): Palladium, metallisch, auf Stahldrahtnetzen, handelsüblicher Katalysator. | | | | | |
| (*2): Erfindungsgemäßer Mn/Co/Ce-MTKS-Sol-Kat, Beschichtungsmasse Beispiel 1 | | | | | |
| (*3): Erfindungsgemäßer Mn/Cu/Ce-MDKS-Sot-Kat, Beschichtungsmasse Beispiel 3. | | | | | |

Es zeigt sich, daß die erfindungsgemäßen katalytischen Zusammensetzungen in der Lage sind, neben den anderen Testsubstanzen, auch Heterocyclen wie Pyrazin und Thiazol abzubauen. Dies ist mit handelsüblichen Palladium-Katalysatoren nicht möglich. Außerdem tritt bei den erfindungsgemäßen katalytischen Zusammensetzungen nach 10 Testzyklen kein Verlust an katalytischer Aktivität auf. Dagegen wird der handelsübliche Palladium-Katalysator durch Heterocyclen wie Thiazol vergiftet, d.h. er verliert mit der Zeit an katalytischer Aktivität.

### Bewertung des Oxidationsvermögens

### [Prüfmethode nach DIN 51 171, "Prüfung des Selbstreinigungsvermögens kontinuierlich selbstreinigender Emaillierungen"]

Auf die zu untersuchenden Proben werden an fünf auf einem Kreis angeordneten Stellen so lange definierte Mengen (je 20-25 mg) Soja- oder Motorenöl aufgetropft, und nach jedem Auftropfen durch eine einstündige Wärmebehandlung bei 250°C verbrannt, bis infolge der Ansammlung unverbrannter Rückstände ein sichtbarer Glanz auftritt. Zur Beurteilung dienen die Anzahlen der Zyklen bis zum Glanzauftritt.

| Beschichtung | Öl | Zahl der Zyklen bis Glanz |
|---|---|---|
| (*1) | Soja | 4-5 |
| (*2) | Soja | 15-17 |
| (*3) | Soja | 13-15 |

| | | |
|---|---|---|
| (*1): Handelsübliches, oxidativ wirkendes Email, enthält Fe/Mn-Oxide | | |
| (*2): Katalytische Zusammensetzung 1 | | |
| (*3): Katalytische Zusammensetzung 1, aber unter Verwendung von Kieselgel 40 als Trägermaterial anstelle von Aluminiumoxid 90 | | |

Die erfindungsgemäßen katalytischen Zusammensetzungen (Schichtdicken zwischen 150-400 Mikrometer) weisen aufgrund der vorhandenen Hohlräume in der Beschichtung eine hohe Saugfähigkeit und damit ein gutes Spreitungsvermögen für Öle auf. Im Gegensatz dazu weisen die glasartigen Emails ein geringes Saug- und Spreitungsvermögen auf.

## Patentansprüche

1. Haushaltsgeräte mit einer katalytischen Zusammensetzung für Desodorisierungs- oder Oxidationszwecke, die eine Beschichtung aus einer Beschichtungsmasse auf einem Substrat umfasst und erhältlich ist durch Aufbringen der Beschichtungsmasse, umfassend (1) ein Polykondensat aus
(A) einem oder mehreren Silanen der allgemeinen Formel (I)
RₐSiX₍₄₋ₐ₎ (I)
worin die Reste R gleich oder verschieden sind und nicht hydrolysierbare Gruppen darstellen, die Reste X gleich oder verschieden sind und hydrolysierbare Gruppen oder Hydroxylgruppen bedeuten und a den Wert 0, 1, 2 oder 3 hat, wobei bei mindestens 50 Stoffmengen-% der Silane a großer 0 ist, oder einem davon abgeleiteten Oligomer,
(B) gegebenenfalls einer oder mehreren Verbindungen von glasbildenden Elementen,
und (2) Teilchen von einem oder mehreren katalytisch wirkenden Übergangsmetalloxiden oder Teilchen, die ein oder mehrere katalytisch wirkende Übergangsmetalloxide auf der Oberfläche aufweisen, wobei das Gewichtsverhältnis von Übergangsmetalloxid-Teilchen zu Polykondensat 10:1 bis 1:10 beträgt, auf das Substrat und thermisches Behandeln der aufgebrachten Beschichtungsmasse, wobei die katalytische Zusammensetzung Bestandteil des Haushaltsgeräts oder einer mit dem Haushaltsgerät verbundenen Einrichtung ist.

2. Haushaltsgerät nach Anspruch 1, **dadurch gekennzeichnet, dass** bei 50 bis 95 Stoffmengen-% der Silane a größer 0 ist.

3. Haushaltsgerät nach Anspruch 1 oder 2, **dadurch gekennzeichnet, dass** das Übergangsmetalloxid ausgewählt ist aus den Oxiden der Metalle La, Ce, Ti, Zr, V, Cr, Mo, W, Mn, Fe, Co, Ni, Cu, Ag und Zn oder aus Mischungen dieser Oxide.

4. Haushaltsgerät nach einem der Ansprüche 1 bis 3 **dadurch gekennzeichnet, dass** der Teilchendurchmesser der Übergangsmetalloxid-Teilchen 10 nm bis 20 µm beträgt.

5. Haushaltsgerät nach einem der Ansprüche 1 bis 4, **dadurch gekennzeichnet, dass** die Schichtdicke der Beschichtung 0,01 bis 500 µm beträgt.

6. Haushaltsgerät nach einem der Ansprüche 1 bis 5, **dadurch gekennzeichnet, dass** das Substrat aus Metall, Metalloxid, Glas, Glaskeramik, Keramik oder porösem Material besteht.

7. Haushaltsgerät nach einem der Ansprüche 1 bis 6, **dadurch gekennzeichnet, dass** die Beschichtungsmasse, gegebenenfalls nach vorherigem Trocknen, in einem Temperaturbereich von 200 bis 700°C behandelt worden ist.

8. Haushaltsgerät nach einem der Ansprüche 1 bis 7, **dadurch gekennzeichnet, dass** in der Beschichtungsmasse zusätzlich anorganische Partikel enthalten sind.

9. Haushaltsgerät nach einem der Ansprüche 1 bis 8, **dadurch gekennzeichnet, dass** die Beschichtung aus der Beschichtungsmasse porös ist.

10. Haushaltsgerät nach einem der Ansprüche 1 bis 9, **dadurch gekennzeichnet, dass** die katalytisch wirkenden Übergangsmetalloxid-Teilchen oder die zusätzlichen anorganischen Partikel einen durchschnittlichen Teilchendurchmesser von mindestens 1 µm aufweisen.

## Claims

1. Domestic appliances comprising a catalytic composition for deodorizing or oxidizing purposes, said catalytic composition comprising a coating of a coating material on a support and being obtainable by applying the coating material, comprising (1) a polycondensate of
(A) one or more silanes of the general formula (I)
Rₐ-Si-X₍₄₋ₐ₎ (I)
in which the radicals R are identical or different and are non-hydrolysable groups, the radicals X are identical or different and are hydrolysable groups or hydroxyl groups and a has the value 0, 1, 2 or 3, with a being greater than 0 for at least 50 mol% of the silanes, or an oligomer derived therefrom,
(B) if desired, one or more compounds of glass-forming elements,
and (2) particles of one or more catalytically active transition metal oxides or particles having one or more catalytically active transition metal oxides on their surface, the weight ratio of transition metal oxide particles to polycondensate being from 10:1 to 1:10, to the support and subjecting the applied coating material to thermal treatment, said catalytic composition representing a component of said domestic appliance or of a device connected with said domestic appliance.

2. Domestic appliance according to Claim 1, **characterized in that** a is greater than 0 for from 50 to 95 mol% of the silanes.

3. Domestic appliance according to Claim 1 or 2, **characterized in that** the transition metal oxide is selected from the oxides of the metals La, Ce, Ti, Zr, V, Cr, Mo, W, Mn, Fe, Co, Ni, Cu, Ag and Zn or mixtures thereof.

4. Domestic appliance according to one of Claims 1 to 3, **characterized in that** the particle diameter of the transition metal oxide particles is from 10 nm to 20 µm.

5. Domestic appliance according to one of Claims 1 to 4, **characterized in that** the thickness of the coating is from 0.01 to 500 µm.

6. Domestic appliance according to one of Claims 1 to 5, **characterized in that** the support is composed of metal, metal oxide, glass, glass ceramic, ceramic or porous material.

7. Domestic appliance according to one of Claims 1 to 6, **characterized in that** the coating material, optionally after drying, has been treated at a temperature within the range of from 200 to 700°C.

8. Domestic appliance according to one of Claims 1 to 7, **characterized in that** the coating material additionally includes inorganic particles.

9. Domestic appliance according to one of Claims 1 to 8, **characterized in that** the coating formed from the coating material is porous.

10. Domestic appliance according to one of claims 1 to 9, **characterized in that** the catalytically active transition metal oxide particles or the additional inorganic particles have an average particle diameter of at least 1 µm.

## Revendications

1. Appareils ménagers munis d'une composition catalytique à effets oxydant et désodorisant, qui comporte un revêtement constitué d'une masse de matériau de revêtement étalée sur un substrat et qui est obtenue par dépôt sur le substrat de cette masse de matériau de revêtement, laquelle comporte
1) un polycondensat constitué
a) d'un ou de plusieurs silanes de formule générale (I) :
RₐSiX₍₄₋ₐ₎ (I)
dans laquelle les restes symbolisés par R, identiques ou différents, sont des groupes non hydrolysables, les restes symbolisés par X, identiques ou différents, sont des groupes hydrolysables ou des groupes hydroxyle, et a vaut 0, 1, 2 ou 3, a étant supérieur à 0 chez au moins 50 % en moles de ces silanes,
ou d'un oligomère dérivé d'un tel silane,
b) et le cas échéant, d'un ou de plusieurs composés d'éléments formateurs de verre,
2) et des particules d'un ou de plusieurs oxydes de métaux de transition dotés d'une activité catalytique, ou des particules portant en surface un ou plusieurs oxydes de métaux de transition dotés d'une activité catalytique,
le rapport pondéral de ces particules d'oxydes de métaux de transition au polycondensat valant de 10/1 à 1/10,
et traitement thermique de la masse de matériau de revêtement déposée, la composition catalytique étant une partie constitutive de l'appareil ménager ou d'un équipement associé à l'appareil ménager.

2. Appareil ménager conforme à la revendication 1, **caractérisé en ce que** a est supérieur à 0 chez 50 à 95 % en moles des silanes.

3. Appareil ménager conforme à la revendication 1 ou 2, **caractérisé en ce que** l'oxyde de métal de transition est choisi parmi les oxydes des métaux La, Ce, Ti, Zr, V, Cr, Mo, W, Mn, Fe, Co, Ni, Cu, Ag et Zn et les mélanges de ces oxydes.

4. Appareil ménager conforme à l'une des revendications 1 à 3, **caractérisé en ce que** le diamètre des particules d'oxydes de métaux de transition vaut de 10 nm à 20 µm.

5. Appareil ménager conforme à l'une des revendications 1 à 4, **caractérisé en ce que** l'épaisseur de la couche de revêtement vaut de 0,01 à 500 µm.

6. Appareil ménager conforme à l'une des revendications 1 à 5, **caractérisé en ce que** le substrat est constitué d'un métal, d'un oxyde de métal, d'un verre, d'une vitrocéramique, d'une céramique ou d'un matériau poreux.

7. Appareil ménager conforme à l'une des revendications 1 à 6, **caractérisé en ce que** la masse de matériau de revêtement a été traitée, le cas échéant après séchage préalable, à une température située dans l'intervalle allant de 200 à 700 °C.

8. Appareil ménager conforme à l'une des revendications 1 à 7, **caractérisé en ce que** la masse de matériau de revêtement contient en outre des particules de matériau inorganique.

9. Appareil ménager conforme à l'une des revendications 1 à 8, **caractérisé en ce que** le revêtement obtenu à partir de la masse de matériau de revêtement est poreux.

10. Appareil ménager conforme à l'une des revendications 1 à 9, **caractérisé en ce que** le diamètre moyen des particules d'oxydes de métaux de transition dotés d'une activité catalytique ou des particules supplémentaires de matériau inorganique vaut au moins 1 µm.
